Europäisches Patentamt

European Patent Office

Office européen des brevets

(19)

(11) Veröffentlichungsnummer: **0 301 341**
**B1**

(12) **EUROPÄISCHE PATENTSCHRIFT**

(45) Veröffentlichungstag der Patentschrift: 09.01.91

(51) Int. Cl.⁵: **C 07 C 15/00, C 07 C 7/148**

(21) Anmeldenummer: **88111497.9**

(22) Anmeldetag: **18.07.88**

(54) **Verfahren zur Abtrennung von reinen Aromaten.**

(30) Priorität: **30.07.87 DE 3725228**

(43) Veröffentlichungstag der Anmeldung:
**01.02.89 Patentblatt 89/05**

(45) Bekanntmachung des Hinweises auf die
Patenterteilung:
**09.01.91 Patentblatt 91/02**

(84) Benannte Vertragsstaaten:
**AT BE CH DE ES FR GB IT LI NL SE**

(56) Entgegenhaltungen:
**Keine Entgegenhaltungen**

(73) Patentinhaber: **BAYER AG**
**D-5090 Leverkusen 1 Bayerwerk (DE)**
(73) Patentinhaber: **EC ERDÖLCHEMIE GMBH**
**Postfach 75 20 02**
**D-5000 Köln 71 (DE)**

(72) Erfinder: **Krämer, Hans-Joachim, Dr.**
**Robert-Koch-Strasse 15**
**D-4047 Dormagen (DE)**
Erfinder: **Schulwitz, Bruno, Dr.**
**An der Ronne 86**
**D-5000 Koeln 40 (DE)**
Erfinder: **Horlitz, Werner**
**Hackhauser-Strasse 32**
**D-4047 Dormagen 1 (DE)**
Erfinder: **Lange, Peter Michael, Dr.**
**Walter-Flex-Strasse 9**
**D-5090 Leverkusen (DE)**
Erfinder: **Mischker, Alfred, Dr.**
**Am Gartenfeld 50**
**D-5068 Odenthal-Holz (DE)**

# EP 0 301 341 B1

**Beschreibung**

Die Erfindung betrifft ein Verfahren zur Abtrennung von reinen aromatischen Kohlenwasserstoffen aus solche enthaltenden Kohlenwasserstoffgemischen durch Extraktion in Gegenwart von Reaktivstoffen, wobei gleichzeitig weitgehende aromatenfreie Raffinate erhalten werden.

Die Ausgangsmaterialien für die erfindungsgemäße Abtrennung von Reinaromaten sind Gemische von aromatischen und nichtaromatischen Kohlenwasserstoffen, aus denen die reinen Aromaten durch einfache thermische Destillation aufgrund ähnlicher Siedelagen nicht abgetrennt werden können. Die Gemische können die Aromaten, beispielsweise Benzol, Toluol und Xylol, sowohl gemeinsam als auch einzeln enthalten. Gemische der genannten Art fallen beispielsweise in Raffinieren und petrochemischen Anlagen an.

Es ist bekannt, daß die Nichtaromaten von den Aromaten durch Extraktion mit selektiven Lösungsmitteln abgetrennt werden können. Als Extraktion sei gleichermaßen die klassische Flüssigphasen-Extraktion wie die Extraktivdestillation verstanden. In beiden Verfahren werden durch ein Lösungsmittel die Aromaten aus dem Kohlenwasserstoff selektiv herausgelöst. Bei der Flüssigphasen-Extraktion erfolgt die Abtrennung des aromatenarmen Raffinats vom aromatenreichen Lösungsmittel durch mechanische Trennung in einem Extraktor; bei der Extraktivdestillation erfolgt die Trennung durch Destillation in eine Kolonne. Die Isolierung des bzw. der Reinaromaten erfolgt in beiden Fällen in einer nachgeschalteten Kolonne (Stripper) durch Destillation des bzw. der Reinaromaten vom Lösungsmittel. Das aromatenarme Lösungsmittel wird aus dem Sumpf des Strippers in den Flüssigphasen-Extraktor bzw. in die Extraktivdestillationskolonne zurückgeführt.

Bei beiden verfahrenstechnischen Varianten der Gewinnung von Reinaromaten aus aromatenhaltigen Kohlenwasserstoffgemischen wird der spezifische Trennaufwand durch folgende Faktoren bestimmt;

a) durch die Aromatenkonzentration im Einsatzgemisch.

b) durch die Aromatenausbeute und

c) durch die Qualitätsanforderungen an den bzw. die Reinaromaten bzw. an das Raffinat.

Setzt man beispielsweise den Fall, daß die Aromatenkonzentration des Einsatzstromes konstant ist und die Qualitätsanforderungen an den (die) Reinaromaten bezüglich der Nichtaromaten und der Lösungsmittelspuren sowie die Qualitätsanforderungen an das Raffinat bezüglich Lösungsmittelspuren und Aromatengehalt ebenfalls gleich bleiben, steigt der Energieaufwand mit der gewünschten Ausbeute an Aromaten. Der optimale verfahrenstechnische Punkt wird sodann durch die Energiekosten einerseits und die aus dem Verkauf bzw. der Weiterverarbeitung der Aromaten und des Raffinats erzielten Erlöse andererseits bestimmt.

Die vorliegende Erfindung ermöglicht es nun, bei gleicher Aromatenausbeute den spezifischen Trennaufwand zu senken, bzw. bei gleichem spezifischen Trennaufwand die Aromatenausbeute su erhöhen, wobei auch Betriebszustände zwischen den genannten Extremwerten, sofern sie wünschenwert sind, eingestellt werden können. Da zukünftig gesetzliche Restriktionen bezüglich des Aromatengehalts im Vergaserkraftstoff zu erwarten sind, ist es notwendig, den Aromatengehalt im aromatenarmen Raffinat soweit wie möglich zu senken, woraus sich gleichzeitig in bevorzugter Weise ein Priorität zu einer erhöhten Aromatenausbeute bei gleichem spezifischen Trennaufwand ergibt.

Es wurde nun ein Verfahren zur Abtrennung von Aromaten aus Kohlenwasserstoffgemischen durch Extraktion (Flüssigphasen-Extraktion bzw. Extraktivdestillation) mit einem selektiven Lösungsmittel gefunden, das durch gekennzeichnet ist, daß man die Extraktion in Gegenwart eines reaktiven Feststoffes durchführt.

Als reaktive Feststoffe für das erfindungsgemäße Verfahren seien solche verstanden, die nur mit bestimmten Komponenten eines zu trennenden Gemisches eine Wechselwirkung eingehen, während sie mit den restlichen Komponenten des Gemisches keine oder nur geringe Wechselwirkungen haben. Als Wechselwirkungen seien in diesem Zusammenhang beispielsweise das Quellverhalten eines Feststoffes mit den in dem Gemisch enthaltenen bestimmten Komponenten, sowie das Elutionsverhalten des mit den geeigneten bestimmten Komponenten gequollenen Feststoffes genannt. Auch elektronische Wechselwirkungen können einen Beitrag zur verbesserten Extraktion in Gegenwart der reaktiven Feststoffe leisten. Werden beispielsweise reaktive Feststoffe an geeignete Stelle in eine Extraktionskolonne eingebracht, ist eine verbesserte Auftrennung möglich, wenn das verwendete Extraktionsmittel in der Lage ist, die angereicherten bestimmten Komponenten von diesen reaktiven Feststoffen zu eluieren. Der reaktive Feststoff kann auch mit dem Lösungsmittel Wechselwirkungen eingehen und zusätzlich eine stationäre Phasen bilden.

Die erfindungsgemäß einzusetzenden reaktiven Feststoffe lassen sich beispielsweise durch eine klassische Perpolymerisation, wie sie im Houben-Weyl Bd. 14/1 S. 133 ff beschrieben ist, leicht herstellen. So erhält man die erfindungsgemäß einzusetzenden Feststoff, wenn man beispielweise Styrol in Gegenwart von 2—85 Gew.-% Divinylbenzol (DVB), gegebenenfalls in Form von technischem DVB und wechselnden Mengen eines Porogens oder technisches DVB allein mit wechselnden Mengen eines Porogens umsetzt. Die geeigneten Porogene sind dem Fachmann bekannt. Man kann zur Erzeugung von makroporösen reaktiven Feststoffen sogenannte Fäll- oder Quellmittel bzw. deren Mischungen einsetzen.

Auch die Verwendung anderer Monomere ist denkbar, wenn dadurch die Wechselwirkungen, wie z.B. das Quellverhalten der so erhältlichen Polymere mit Aromaten, erhalten bleibt. Bevorzugt sind jedoch Grundharze für Ionenaustauscher auf Basis Styrol/DVB, nicht zuletzt deshalb, weil sie technisch in entsprechenden Mengen zu Verfügung stehen. Die reaktiven Feststoffe können in Gelform oder in makroporöser Form zur Anwendung kommen. Meistens wird jedoch der Einsatz von makroporösen Grundkörpern bessere Trennergebnisse erbringen. Der Vernetzungsgrad der Grundkörper kann zwischen 2 und 85% betragen. Bekanntlich nimmt mit höherer Vernetzung die Oberfläche der makroporösen Grundkörper zu, so daß somit ein intensiverer Kontakt zwischen Aromaten und reaktivem Feststoff möglich ist. Aus diesem Grund sind Grundkörper, die 40—85 Gew.-% technisches DVB enthalten, besonders bevorzugt.

Als Beispiel sie die Extraktion in der bevorzugten Form einer Extraktivdestillation wie folgt beschrieben: In einem Dampfstrom, bestehend aus aliphatischen Kohlenwasserstoffen und Aromaten, gehen die aromatischen Komponenten mit reaktiven Feststoffen auf der Basis eines Styrol-Divinylbenzol-Polymerisats starke Wechselwirkungen ein, während die aliphatischen Kohlenwasserstoffe praktisch keine Wechselwirkungen mit den genannten reaktiven Feststoffen entfalten. Das auch hier im Gegenstrom eingesetzt selektive Lösungsmittel eluiert, zusätzlich zum bereits bekannten Effekt der Extraktivdestillation, Anteile der Aromaten aus den Reaktivstoffen. Damit wird die Trennleistung bei gleichem Energieaufwand deutlich erhöht.

Für den Fall der klassischen Flüssigphasen-Extraktion werden solche reaktive Feststoffe in die Beruhigungskammern eines Extraktors eingebracht.

Es hat sich als vorteilhaft erwiesen, die reaktiven Feststoffe beispielsweise innerhalb von tetraedrisch geformten Drahtnetzen zu fixieren und diese Drahtnetze als Füllkörper in Extraktivdestillationskolonnen bzw. Beruhigungskammern von Extraktoren einzubringen. Vorteilhafterweise werden die tetraedrisch geformten Drahtnetze nur teilweise mit dem reaktiven Feststoff gefüllt, um Quellvorgänge zu gestatten.

In einem weiteren Beispiel lassen sich aus Drahtnetze mit den reaktiven Feststoffen belegen und anschließend zusammenrollen, so daß auch hierdurch wieder geeignete Füllkörper entstehen. Anstelle der Drahtnetze können solche reaktiven Feststoffe auch in poröse Kunststoffmatten eingerollt werden, die dann als Füllkörper dienen können. Es ist weiterhin denkbar, handelsübliche Füllkörper, wie Raschigringe oder Berlsättel, mit reaktiven Feststoffen so zu belegen, daß diese reaktiven Feststoffe auf den Füllkörpern fixiert sind, woraufhin solche belegten Füllkörper in der beschriebenen Weise eingesetzt werden.

Als selektive Lösungsmittel können beispielsweise eingesetzt werden: N-Methyl-pyrrolidon, N-Formylmorpholin, Sulfolan, Acetonitril.

Die bei der Extraktion (klassische Flüssigphasen-Extraktion bzw. Extraktivdestillation) einzustellenden übrigen Verfahrensparameter wie Temperatur, Druck, Durchflußgeschwindigkeit, u.a. sind dem Fachmann bekannt.

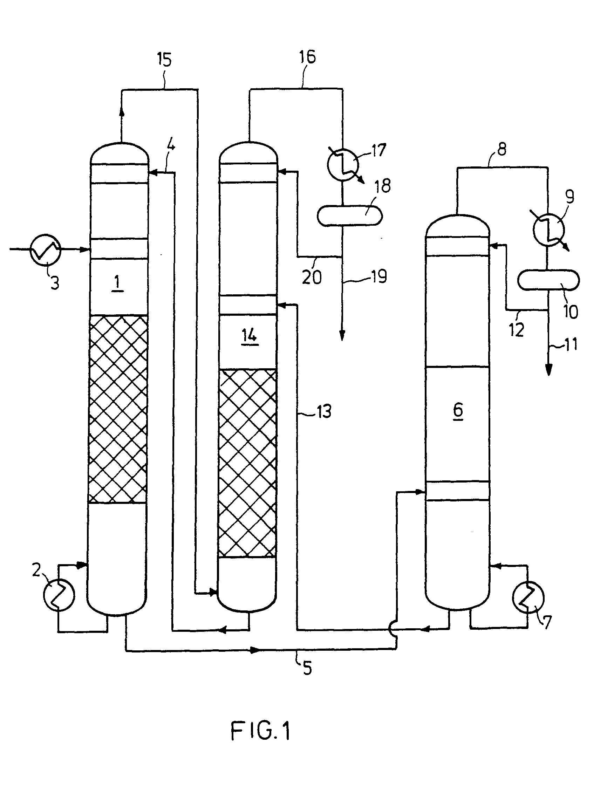

Ebenfalls dem Fachmann bekannt ist die Verschaltung des eigentlichen Extraktionsapparates (Extraktor bzw. Extraktivdestillationskolonne) mit einem Stripper. Für den Fall einer Extraktivdestillation sei das beispielhaft anhand der beiliegenden Abbildung beschrieben:

Einer Extraktivdestillationskolonne (1) mit Sumpfumlaufheizung (2) wird im oberen Teil ein Benzolschnitt über die Leitung (3) zugeführt. Das Extraktivlösungsmittel wird (1) über die Leitung (4) oberhalb von (3) zugeführt. Ein Sumpfstrom wird über die Leitung (5) der Stripperkolonne (6) mit Sumpfumlaufheizung (7) zugeführt und dort in Reinbenzol und regeneriertes Extraktivlösungsmittel aufgetrennt. Das Reibenzol wird als Kopfstrom über die Leitung (8), den Kondensator (9), die Auffangwanne (10) und die Leitung (11) entnommen. Leitung (12) dient der Einstellung des Rücklaufverhältnisses. Das regenierte Extraktivlösungsmittel wird als Sumpfstrom über die Leitung (13) einer Verstärkungskolonne (14) für die Extraktivdestillation zugeführt. (13) wird am unteren Ende mit dem Kopfstrom von (1) über die Leitung (15) beschickt. Das aromatenfreie Raffinat wird (14) als Kopfstrom über die Leitung (16), den Kondensator (17), die Auffangwanne (18) und die Leitung (19) entnommen. Die Leitung (20) dient der Einstellung des Rücklaufverhältnisses. (14) und gegebenenfalls auch (1) sind erfindungsgemäß mit reaktiven Feststoffen ausgerüstet.

Beispiel

Im Labor wurden in einem kontinuierlichen Versuch 580 g/l eines Benzolschnittes (ca. 80 Gew.-% Benzol und ca. 20 Gew.-% Nichtaromaten) mit Hilfe des Lösungsmittels N-Methyl-pyrrolidon (NMP) in einer Extraktivdestillationskolonne und einer nachgeschalteten Stripperkolonne in Reinbenzol und ein benzolarmes Raffinat (B-Raffinat) getrennt. Die Extraktivdestillationskolonne hatte eine theoretische Bodenzahl von 48. Die Produktführung geschah in der in der Abbildung gezeigten Weise.

Die Extraktivdestillationskolonne war mit tetraedrischen Netzfüllkörpern gepackt, wobei die Netzfüllkörper makroporöses Styrol-Divinylbenzol-Perlpolymerisat enthielten. Die Netzfüllkörper wurden in den Bereich der Extraktionszone, also unterhalb der NMP-Aufgabe eingebracht, wie es die Abbildung zeigt.

In einem Vergleichsversuch wurden ebenfalls Netzfüllkörper eingebracht, die jedoch schwach-sauer, poröse Ionenaustauscher, auf Basis Lewatit CNP 80 enthielten.

Es wurden folgende Ergebnisse erhalten:

| | erfindungsgemäßer Versuch | Vergleichs- versuch |
|---|---|---|
| NMP-Menge zu Benzolschnitt | 3:1 | 3:1 |
| Nichtaromaten im Benzol | 210 ppm | 150 ppm |
| Benzol im B-Raffinat | 0,7 Gew.-% | 5,1 Gew.-% |
| Benzolausbeute | 99,8% | 98,8% |

**Patentansprüche**

1. Verfahren zur Abtrennung von Aromaten aus Kohlenwasserstoffgemischen durch Extraktion (Flüssigkphasen-Extraktion bzw. Extraktivdestillation) mit einem selektiven Lösungsmittel, dadurch gekennzeichnet, daß man die Extraktion in Gegenwart eines reaktiven Feststoffes durchführt.

2. Verfahren nach Anspruch 1, dadurch gekennzeichnet, daß man den reaktiven Feststoff als reaktive Füllkörper in eine Extraktivdestillationskolonne bzw. in die Beruhigungskammern eines Extraktors einbringt.

3. Verfahren nach Anspruch 2, dadurch gekennzeichnet, daß als reaktive Füllkörper geschlossene Drahtnetze oder zusammengerollte poröse Kunststoffmatten dienen, in die reaktiver Feststoff eingebracht worden ist.

4. Verfahren nach Anspruch 1, dadurch gekennzeichnet, daß man als reaktive Feststoffe Grundharze für Ionenaustauscher ohne die ionenaustauschenden Gruppen verwendet.

5. Verfahren nach Anspruch 4, dadurch gekennzeichnet, daß man als reaktive Feststoffe Styrol-Divinylbenzol-Perlpolymerisate verwendet.

6. Verfahren nach Anspruch 5, dadurch gekennzeichnet, daß Styrol-Divinylbenzol-Perlpolymerisate in makroporöser Form verwendet werden.

7. Verfahren nach Anspruch 5, dadurch gekennzeichnet, daß Styrol-Divinylbenzol-Polymerisate mit 2—85 Gew.-% Divinylbenzol, besonders bevorzugt von 40 bis 85 Gw.-% DVB, verwendet werden.

**Revendications**

1. Procédé pour séparer les hydrocarbures aromatiques de mélanges d'hydrocarbures par extraction (extraction en phase liquide ou distillation extraktive) avec un solvant sélectif, caractérisé en ce qu'on effectue l'extraction en présence d'une substunce solide réactive.

2. Procédé selon la revendication 1, caractérisé en ce qu'on introduit la substance solide réactive sous forme de corps de remplissage réactifs dans une colonne de distillation extractive ou dans les chambres de repos d'un extracteur.

3. Procédé selon la revendication 2, caractérisé en ce qu'on utilise comme corps de remplissage réactifs des treillis de fil métallique fermés ou des mats de matière synthétique poreuse enroulés dans lesquels la substance solide réactif a été introduite.

4. Procédé selon la revendication 1, caractérisé en ce qu'on utilise comme substance solide réactive une résine de base pour échangeurs d'ions sans les groupes échangeurs d'ions.

5. Procédé selon la revendication 4, caractérisé en ce qu'on utilise comme substance solide réactive un produit de polymérisation en perles de styrène-divinylbenzène.

6. Procédé selon la revendication 5, caractérisé en ce que le produit de polymérisation en perles de styrène-divinylbenzène est utilisé sous forme macroporeuse.

7. Procédé selon la revendication 5, caractérisé en ce qu'on utilise un produit de polymérisation de styrène-divinylbenzène avec 2 à 85% en oids de divinylbenzène, de préférence 40 à 85% en poids de DVB.

**Claims**

1. Process for removing aromatics from hydrocarbon mixtures by extraction (liquid-phase extraction or extractive distillation) with a selective solvent, characterized in that the extraction is carried out in the presence of a reactive solid.

2. Process according to Claim 1, characterized in that the reactive solid is introduced as reactive packing elements into an extractive-distillation column or into the contact chambers of an extractor.

3. Process according to Claim 2, characterized in that the reactive packing elements used are closed wire gauzes or rolled-up porous plastic mats into which the reactive solid has been introduced.

4. Process according to Claim 1, characterized in that the reactive solids used are base resins for ion exchangers without the ion-exchanging groups.

**EP 0 301 341 B1**

5. Process according to Claim 4, characterized that the reactive solids used are styrene-divinylbenzene bead polymers.

6. Process according to Claim 5, characterized in that the styrene-divinylbenzene bead polymers are used in macroporous form.

7. Process according to Claim 5, characterized in that the styrene-divinylbenzene polymers used contain 2—85% by weight of divinylbenzene, particularly preferably 40 to 85% by weight of DVB.

FIG.1